# EUROPEAN PATENT APPLICATION

(11) **EP 2 548 571 A1**
(43) Date of publication of application: **23.01.2013**
(21) Application number: 11305959.6
(22) Date of filing: 22.07.2011
(51) Int. Cl.: A61K 39/00

(54) **Compositions having means for targeting at least one antigen to dendritic cells**

(71) Applicant: Institut Curie, 75005 Paris (FR); Centre National de la Recherche Scientifique, 75794 Paris Cedex 16 (FR); INSTITUT NATIONAL DE LA SANTE ET DE LA RECHERCHE MEDICALE, 75654 Paris Cedex 13 (FR); ASSISTANCE PUBLIQUE, HOPITAUX DE PARIS, 75004 Paris (FR); Université René Descartes (Paris V), 75006 Paris (FR)
(72) Inventor: The designation of the inventor has not yet been filed
(74) Representative: Vaillant, Jeanne

(57) **Abstract**

A composition that can be used as a vaccine containing means for targeting at least one antigen to dendritic cells and as adjuvants a granulocyte macrophage colony stimulating factor and a CpG oligodeoxynucleotide and/or a CpG-like oligodeoxynucleotide. This composition can used to treat cancers, infectious diseases caused by bacterial, viral, fungal, parasitic or protozoan infections, allergies and/or autoimmune diseases.

## Description

### Field of the Invention

The present invention relates to a composition that can be used as a vaccine containing means for targeting at least one antigen to dendritic cells. Granulocyte macrophage colony stimulating factor and a CpG oligodeoxynucleotide and/or a CpG-like are used as adjuvants. In one aspect this composition can be used to treat cancers, infectious diseases caused by bacterial, viral, fungal, parasitic or protozoan infections, allergies and/or autoimmune diseases in any warm blooded animal.

### Background of the Invention

A vaccine is a biological preparation injected into the body that produces antibodies and provides immunity against a disease. Vaccines can be prophylactic or therapeutic. Vaccines offer potential advantages with respect to the ease in which they can be manufactured, the low cost of production and the methods in which they are administered. Development of therapeutic vaccines is a challenge since in order to be effective vaccines must elicit a specific and strong CD8⁺ T cell response.

Dendritic cells (DC) are highly specialized antigen-presenting cells (APC) that control a spectrum of immune responses. Dendritic cells can mobilize several immune resistance mechanisms such as CD8⁺T cells, cytotoxic T cells, CD4⁺ helper T cells, natural killer (NK) cells and natural killer T(NKT) cells. Each of these lymphocytes has the capacity to recognize and kill diseased cells, as well as releasing protective cytokines such as IFN-γ and CD4 T cells. Dendritic cells also provide help and maintenance of CD8⁺ cytolytic cells, while Nk and NKT cells can eliminate molecules that hinder presentation on MHC class I, thus escaping CTL recognition. Thus, it is quite important in immunization that the antigen is delivered to dendritic cells to achieve antigen presentation *in vivo* as well as dendritic cell maturation to differentiate the cells such that they do not induce tolerance by different mechanisms.

The transition of dendritic cells from antigen processing to antigen presenting cells is often accompanied by increased expression of class I and class II Major histocompatibility proteins (MHC). Activated and mature dendritic cells induces specific T-cell immunity and resistance to tumors and other diseased states.

Many carriers are known in the art to deliver various antigens to warm blooded animals such as liposomes, microparticles and nanoparticles, as well as toxin vectors and monoclonal and polyclonal antibodies. However, many of these carriers do not target dendritic cells and do not have sufficient adjuvant properties to stimulate a sufficient immune response.

The B subunit of Shiga toxin, a toxin vector, is known to be a universal carrier that can be used to target directly or indirectly the Gb3 receptor. U.S. Patent No. 6,613,882 describes a chimeric polypeptide of the formula B-X, where B is the B subunit of Shiga toxin or functional equivalent thereof and X is a polypeptide of therapeutic significance. U.S. Patent 7,632,514 B2 describes a carrier having the formula STxB-Z (n)-Cys-X where STxB is the B subunit of Shiga toxin Z is an amino acid linker without sulfhydryl groups and n is 0, 1, 2 or a polypeptide and Cys is Cysteine.

U.S. Patent application No: 0100266672 describes a vaccine composition comprising the B subunit of Shiga toxin or an immunologically functional equivalent thereof which is able to bind the Gb3 receptor and is complexed with at least a first antigen and further comprising a second antigen and an adjuvant selected from the group of metal salts, oil and water emulsions, Toll like receptor ligands, saponins and combinations thereof.

Although various types of carriers are used as vaccines there is still a need in this art to improve the immunogenicity and hence the effectiveness of vaccines to treat various disease states.

### Summary of the Invention

The present invention provides a composition comprising means for targeting at least one antigen to dendritic cells said at least one antigen being combined with said means and an adjuvant comprising a granulocyte macrophage colony stimulating factor (GM-CSF) and a CpG oligodeoxynucleotide and/or a CpG-like oligodeoxynucleotide.

In another aspect, the present invention provides a composition comprising a carrier that targets dendritic cells combined with at least one antigen and an adjuvant comprising a granulocyte macrophage colony stimulating factor (GM-CSF) and a CpG oligodeoxynucleotide and/or a CpG-like oligodeoxynucleotide. In this aspect the carrier can be any carrier that targets dendritic cells such as liposomes complexed with antibodies, microparticles complexed with antibodies and nanoparticles complexed with antibodies, toxin carriers that target dendritic cells or monoclonal or polyclonal antibodies that target dendritic cells. Fragments of the monoclonal or polyclonal antibodies can also be used as long as they maintain their targeting capacity.

In another aspect, the means for targeting the at least one antigen to dendritic cells is an anti-DEC205 antibody, preferentially CD205, NLDC-145, fragments thereof retaining their dendritic cell targeting capabilities, and mixtures thereof.

In yet another aspect, the present invention provides a composition comprising the B subunit of Shiga toxin or an immunologically functional equivalent thereof, which targets dendritic cells and is combined with at least one antigen and an adjuvant comprising a granulocyte macrophage colony stimulating factor (GM-CSF) and a CpG oligodeoxynucleotide and/or a CpG-like oligodeoxynucleotide.

In the composition of the present invention the means for targeting dendritic cells or the carrier which targets dendritic cells, toxin carriers that target dendritic cells or monoclonal or polyclonal antibodies that target dendritic cells, the B subunit of Shiga toxin or immunologically functional equivalents thereof which targets dendritic cells are combined with at least one antigen by covalent binding or by electrostatic or hydrophobic interaction or as a fusion protein.

The B subunit of Shiga toxin or said immunologically equivalent thereof combined to said at least one antigen through chemical coupling via a Cysteine group or a Z(n)-Cys group wherein Z is an amino acid devoid of a sulfydryl group and n is 0, 1 or a polypeptide is another aspect of the invention.

The at least one antigen can be a cancer antigen, an antigen from infectious diseases such as a bacterial antigen, a viral antigen, a fungal antigen, a parasitic antigen or a protozoan antigen, an autoimmune antigen, an allergy antigen and mixtures thereof. The composition can thus be used to treat various medical disease states in warm blooded animals.

A composition comprising means for targeting dendritic cells combined with at least one antigen and an adjuvant comprising a granulocyte macrophage colony stimulating factor (GM-CSF) and a CpG oligodeoxynucleotide and/or a CpG-like oligodeoxynucleotide for activating cytotoxic T lymphocytes is also another embodiment of the invention.

A composition comprising a carrier for targeting dendritic cells combined with at least one antigen or toxin carriers for targeting dendritic cells combined to at least one antigen or monoclonal or polyclonal antibodies combined with at least one antigen that target dendritic cells and an adjuvant comprising a granulocyte macrophage colony stimulating factor (GM-CSF) and a CpG oligodeoxynucleotide and/or a CpG-like oligodeoxynucleotide for activating cytotoxic T lymphocytes is also another embodiment of the invention.

A composition comprising the B subunit of Shiga toxin or an immunologically functional equivalent thereof which targets dendritic cells and is combined with at least one antigen and an adjuvant comprising a granulocyte macrophage colony stimulating factor (GM-CSF) and a CpG oligodeoxynucleotide and/or a CpG-like oligodeoxynucleotide for activating cytotoxic T lymphocytes is also another embodiment of the invention.

The compositions as described herein are used as a drug, preferentially as a vaccine in yet another aspect of the invention. Thus, the compositions as described herein can be used for treating cancers, infectious diseases caused by bacteria, viruses, fungus, parasite or protozoans, allergies and/or autoimmune diseases. They also can be used for treating breast cancer.

In yet another aspect the present invention provides a composition for the manufacture of a medicament for vaccinating warm blooded animals comprising means for targeting dendritic cells combined with at least one antigen or a carrier for targeting dendritic cells combined with at least one antigen or toxin carriers that target dendritic cells combined with at least one antigen or monoclonal or polyclonal antibodies combined with at least one antigen that target dendritic cells and an adjuvant comprising a granulocyte macrophage colony stimulating factor (GM-CSF) and a CpG oligodeoxynucleotide and/or a CpG-like oligodeoxynucleotide.

In yet another aspect the present invention provides a composition for the manufacture of a medicament for vaccinating warm blooded animals comprising the B subunit of Shiga toxin or an immunologically functional equivalent thereof which targets dendritic cells and is combined with at least one antigen and an adjuvant comprising a granulocyte macrophage colony stimulating factor (GM-CSF) and a CpG oligodeoxynucleotide and/or a CpG-like oligodeoxynucleotide.

A composition for the manufacture of a medicament to treat cancer, infectious diseases caused by bacterial, viral, fungal, parasitic or protozoan infections, allergies and autoimmune diseases in warm blooded animals comprising means for targeting dendritic cells combined with at least one antigen or a carrier for targeting dendritic cells combined with at least one antigen or toxin carriers that target dendritic cells combined with at least one antigen or monoclonal or polyclonal antibodies combined to at least one antigen that target dendritic cells and an adjuvant comprising a granulocyte macrophage colony stimulating factor (GM-CSF) and a CpG oligodeoxynucleotide and/or a CpG-like oligodeoxynucleotide is yet another embodiment of the present invention.

A composition for the manufacture of a medicament to treat cancer, infectious diseases caused by bacterial, viral, fungal, parasitic or protozoan infections, allergies and autoimmune diseases in warm blooded animals comprising the B subunit of Shiga toxin or an immunologically functional equivalent thereof which targets dendritic cells and is combined with at least one antigen and an adjuvant comprising a granulocyte macrophage colony stimulating factor (GM-CSF) and a CpG oligodeoxynucleotide and/or a CpG-like oligodeoxynucleotide is yet another embodiment of the present invention.

A composition for the manufacture of a medicament to treat breast cancer comprising means for targeting dendritic cells or a carrier that targets dendritic cells, or toxin carriers that target dendritic cells combined with at least one antigen or monoclonal or polyclonal antibodies combined with at least one antigen that target dendritic cells or the B subunit of Shiga toxin or an immunologically functional equivalent of the B subunit of Shiga toxin which targets dendritic cells and is combined with an HER-2/neu antigen and an adjuvant comprising a granulocyte macrophage colony stimulating factor (GM-CSF) and a CpG oligodeoxynucleotide and/or a CpG-like oligodeoxynucleotide is yet another embodiment of the present invention.

A kit for vaccination comprising the compositions as described herein and a pharmaceutically acceptable vehicle is another embodiment of the present invention. This vaccine is suitable for simultaneous, sequential or separate administration to a warm blooded animal.

In another aspect the invention provides a method for activating cytotoxic T lymphocytes said method comprising administering to a warm blooded animal in need of such activation a composition comprising means for targeting dendritic cells or a carrier that targets dendritic cells or toxin carriers that target dendritic cells combined with at least one antigen or monoclonal or polyclonal antibodies combined with at least one antigen that target dendritic cells or the B subunit of Shiga toxin or an immunologically functional equivalent of the B subunit of Shiga toxin which targets dendritic cells combined with at least one antigen and an adjuvant comprising a granulocyte macrophage colony stimulating factor (GM-CSF) and a CpG oligodeoxynucleotide and/or a CpG-like oligodeoxynucleotide.

A method for treating cancers or infectious diseases caused by bacterial, viral, fungal, parasitic or protozoan infections, allergies and autoimmune diseases in a warm blooded animal comprising administering to a warm blooded animal in need of such treatment, a composition comprising means for targeting dendritic cells combined with at least one antigen or a carrier that targets dendritic cells combined with at least one antigen or toxin carriers that targets dendritic cells combined with at least one antigen or monoclonal or polyclonal antibodies combined with at least one antigen that target dendritic cells or the B subunit of Shiga toxin combined with at least one antigen or an immunologically functional equivalent of the B subunit of Shiga toxin which targets dendritic cells and is combined with at least one antigen and an adjuvant comprising a granulocyte macrophage colony stimulating factor (GM-CSF) and a CpG oligodeoxynucleotide and/or a CpG-like oligodeoxynucleotide is still yet another aspect of the present invention.

Yet another method for treating breast cancer is also provided comprising administering to a mammal in need of such breast cancer treatment a composition comprising means for targeting dendritic cells combined with an HER-2/neu antigen or a carrier that targets dendritic cells combined with an HER-2/neu antigen or toxin carriers combined with an HER-2/neu antigen or monoclonal or polyclonal antibodies combined with an HER-2/neu antigen that target dendritic cells or the B subunit of Shiga toxin or an immunologically functional equivalent of the B subunit of Shiga toxin which targets dendritic cells and is combined with an HER-2/neu antigen and an adjuvant comprising a granulocyte macrophage colony stimulating factor (GM-CSF) and a CpG oligodeoxynucleotide and/or a CpG-like oligodeoxynucleotide is still yet another aspect of the present invention.

Other aspects and embodiments are set forth below, or will readily arise from the following description of the preferred embodiments.

### Brief Description of the Drawings

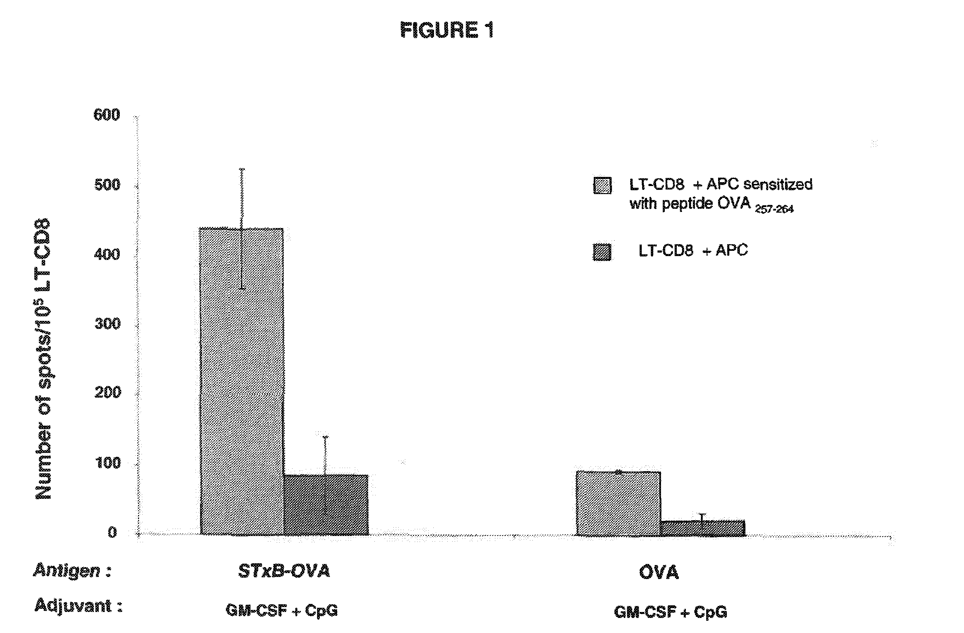

**Fig. 1** is a bar graph showing the CTL induction of LT-CD8 anti-OVA after vaccination in mice with the B subunit of Shiga toxin-OVA ₂₅₇-₂₆₄ or OVA ₂₅₇-₂₆₄ alone in association with 20 µg GM-CSF/CpG in an Elispot assay. These bar graphs show the mean of four mice by group ± standard deviation.

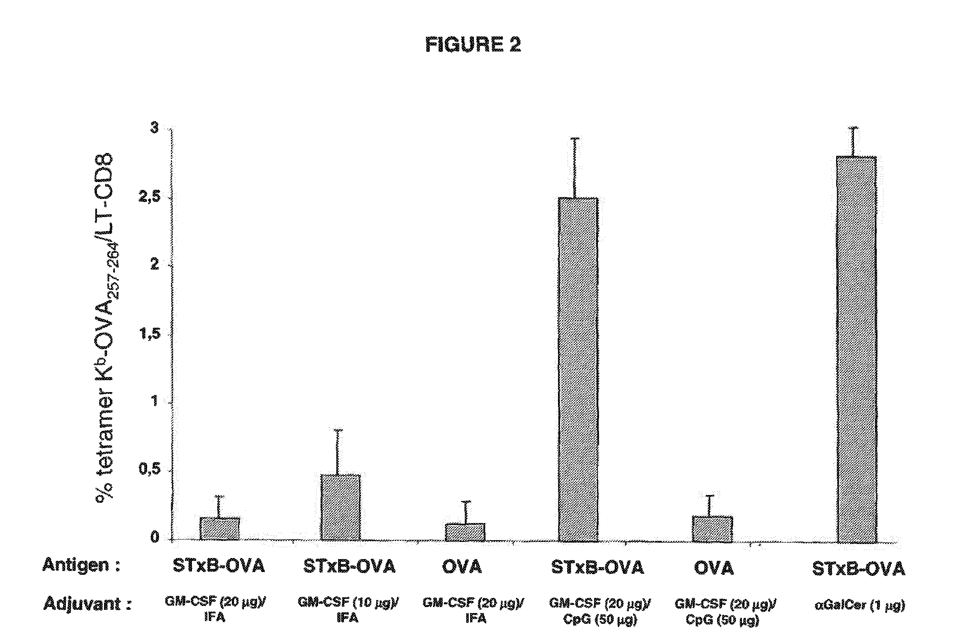

**Fig. 2** is a bar graph showing a comparative analysis in the CTL induction of anti-OVA after mice were vaccinated with the B subunit of Shiga toxin-OVA₂₅₇-₂₆₄ or OVA₂₅₇₋₂₆₄ alone in associate with different adjuvants of αGal Cer, GM-CSF/IFA or GM-CSF/CpG at various concentrations by tetramer analysis. The induction of the tetramer K^{b}-OVA₂₅₇-₂₆₄ was measured and the mean ± standard deviation is shown.

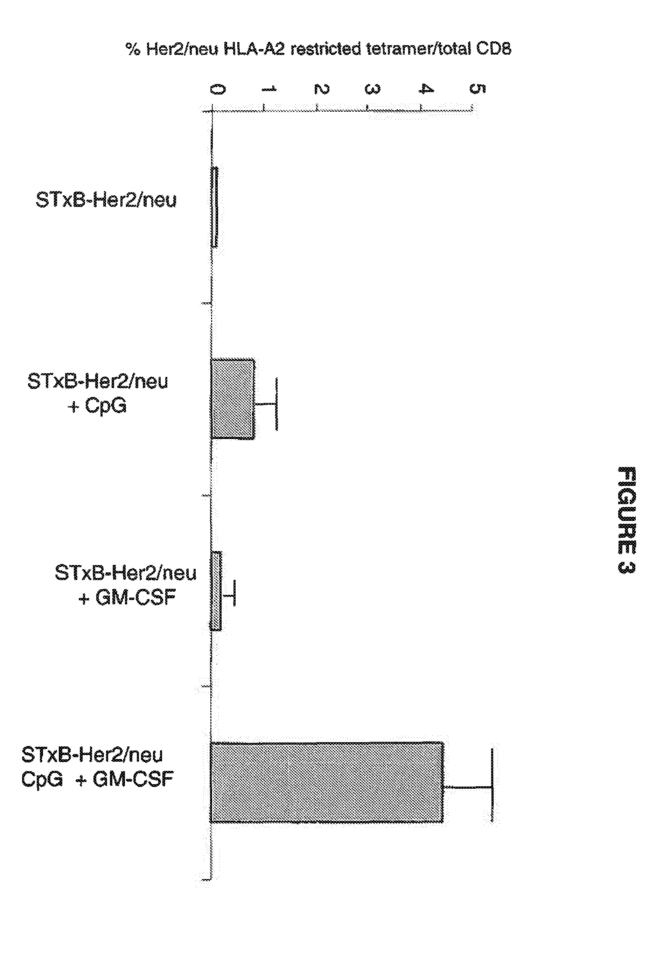

**Fig. 3** is a bar graph showing the synergy between the use of GM-CSF and CpG as an adjuvant with the B subunit of Shiga toxin using tetramer analysis of Her2/neu HLA-A2 restricted tetramer in mice. The mice were immunized with the B subunit of Shiga toxin coupled to the HER-2/neu peptide either alone or with a mixture if CpG (50 µg) or GM-CSF (20 µg) or the combination of GM-CSF and CpG in the same amounts.

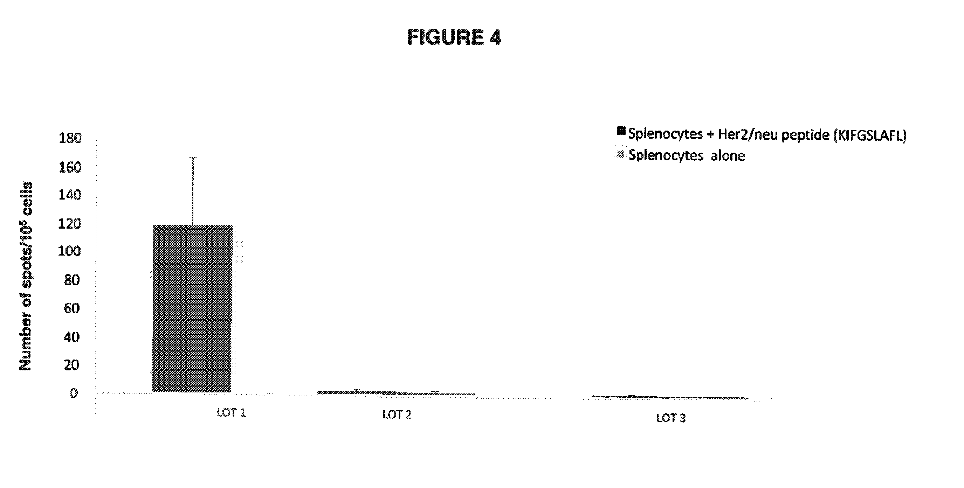

**Fig. 4** is a bar graph showing the results of CTL induction in mice via Elispot assay of the B subunit of Shiga toxin coupled to a peptide IBC002 (RRARKIFGSLAFL (SEQ ID NO: 1)) corresponding to the HER-2/neu peptide (KIFGSLAFL (SEQ ID NO: 2) restricted by HLA-A2 and a sequence (RRAR (SEQ ID NO: 3)) or the peptide IBC002 that is non vectorized or the natural Her2/neu peptide without a flanking sequence in association with 20 µg GM-CSF. 50 µg of CpG was administered the next day. A second immunization without any adjuvant was performed at day 14.

### Description of the Preferred Embodiments

As used herein the abbreviation "APC" means antigen presenting cells, which are cells that display foreign antigen and complexes with the major histocompatibility complex (MHC) on its surface where they can interact with T cell receptors.

The abbreviation "GM-CSF," as used herein, means granulocyte macrophage colony stimulating factor and in the context of the present invention is used as an adjuvant.

As used herein the abbreviation "IFA" means incomplete Freunds Adjuvant.

The abbreviation "CTL" means cytotoxic T lymphocytes, which are lymphocytes that kill other target cells. Most CTL's belong to the CD8⁺ subset of T cells, use the αβT-cell receptor for antigen (TCR), recognize antigens in the groove of class I MHC and if they encounter on a dendritic cell, an antigen/MHC for which their TCR is specific, they enter the cell cycle and differentiate into "killer cells."

The abbreviation "MHC" means major histocompatibility complex, as used herein, and is the immune system's mechanism for displaying peptide antigens to T lymphocytes.

The term "adjuvant" as used herein means any substance that helps or enhances the pharmacological effect of a drug or a vaccine or increases the ability of an antigen to stimulate the immune system.

As used herein "CpG oligodeoxynucleotides" are short DNA sequences bearing unmethylated CpG motifs that bind to the Toll-like receptor 9 (TLR9). TLR is a receptor expressed on B cells and plasmacytoid dendritic cells causing the up regulation of MHC and other stimulatory molecules, which in turn results in more potent APC mediated T cell stimulation. Examples of CpG oligodeoxynucleotides include ODN 2006, ODN D35, ODN 1018 ISS, ODN 1758, ODN 1826, ODN 2216, ODN 2007, ODN 1668, ODN 1720, ODN 2006, ODN 2041, OSN 7909, CpG-28 and the like.

"Combined", as used herein, encompasses all possible means known to those skilled in the art to physically associate a means for targeting at least one antigen to dendritic cells with said at least one antigen. In particular, such term refers to the at least one antigen being chemically coupled to the means for targeting dendritic cells or at least one antigen that is genetically fused to the means for targeting dendritic cells or associated via electrostatic or hydrophobic interaction or any other interaction. Thus, the at least one antigen is physically associated with the means for targeting dendritic cells or the carrier for targeting dendritic cells or toxin carriers for targeting dendritic cells such as the B subunit of Shiga toxin or polyclonal and monoclonal antibodies which target dendritic cells via an electrostatic or hydrophobic interaction or can be covalently linked either chemically or through a fusion protein or linked via a cysteine residue as described in U.S. Patent 7,632,514.

The term "warm blooded animals" as used herein includes birds and mammals.

Examples of birds that can be treated with the compositions and methods of the invention include blue birds, cardinals, doves, eagles, geese, turkeys, chickens, hens, ducks, quails, herons, sparrows, woodpeckers, owls, parrots and the like.

The term "mammal" encompasses any of various warm-blooded vertebrate animals of the class Mammalia, including humans, characterized by a covering of hair on the skin and, in the female, milk-producing mammary glands for nourishing the young. The present invention is not limited to treating humans, but also encompasses veterinary applications, especially since it is well known that animals also can have different medical pathologies.

Examples of mammals that can be treated with the compositions and methods of the present invention include humans, domestic animals such as dogs and cats, horses, mice, goats, deer, cows, rabbits, zoo animals, bears, monkeys, apes, elks, bison, although this invention may be applied to other mammalian species as well.

The abbreviation "LT-CD8, "as used herein, means CD8⁺ T lymphocytes, which are cytotoxic T lymphocytes that monitor the cells of the body and destroy any cells that express foreign antigen fragments in the MHC class pathway.

"Dendritic cells" as used herein means any type of dendritic cells including plasmacytoid dendritic cells, CD8⁺ dendritic cells, CD8- dendritic cells, myeloid dendritic cells, inflammatory dendritic cells, Tip dendritic cells and Langerhans cells. Thus, dendritic cells include migratory dendritic cells such as Langerhans cells and dermal dendritic cells, lymphoid tissue resident dendritic cells such as thymic and splenic dendritic cells and inflammatory dendritic cells.

By "means for targeting dendritic cells" includes any means such as liposomes complexed with antibodies, biomaterials such as nanoparticles complexed with antibodies, microparticles complexed with antibodies and toxin vectors. In another aspect antibodies that target dendritic cells can be complexed directly to the at least one antigen. The antibodies may be monoclonal or polyclonal antibodies that target dendritic cell receptors. Examples of antibodies that can target antigens to dendritic cells include those antibodies that target the mannose receptor, the Fcγ receptor, the asialoglycoprotein receptor, blood DC antigen 2 (BDCA-2), Clec9A, Clec12A, CD11c, CD8, cDC, DCIR-2,FIRE, CIRE, dectin-1, dectin-2, DC-SIGN, L-SIGN, MANR, MMR, MGL, CD23, CD69, CD94, Ly-49 and NKG2. Thus any anti-mannose receptor antibodies, anti-Clec9A antibodies, anti-Clec12A antibodies, anti-CD11c antibodies, anti-CD8 antibodies, anti-cDC antibodies, anti- DCIR-2 antibodies, anti-FIRE antibodies, anti-CIRE antibodies, anti-dectin-1 antibodies, anti-dectin-2 antibodies, anti-DC-SIGN antibodies, anti-L-SIGN antibodies, anti-MANR antibodies, anti- MMR antibodies, MGL anti-antibodies, anti-CD23 antibodies, anti-CD69 antibodies, anti-CD94 antibodies, anti-Ly-49 antibodies and anti- NKG2 antibodies can be used in the compositions and methods of the present invention. Fragments of these antibodies can also be used as long as they retain their targeting capabilities to dendritic cells.

By "immunologically functional equivalent thereof" with respect to the B subunit of Shiga toxin embodiment is meant that the toxin acts immunogenically like the B subunit of Shiga toxin and can bind to Gb3 cells and/or they can internalize the at least one antigen such that the at least one antigen can be presented to the MHC class I pathway or the MHC class I and class II pathways on the same antigen presenting cells. Furthermore, the immunologically functional equivalents can target dendritic cells.

"Treating," as used herein means giving medical care or attention to a warm blooded animal and/or the combating of a disease especially via vaccines which can be therapeutic vaccines or prophylactic vaccines.

By "CpG-like" oligodeoxynucleotide means that the oligodeoxynucleotide has the same interior motif of a high quantity of nucleotides G and C next to one another, but can differ in the exterior motifs, which are not G and C nucleotides and is recognized by toll-like receptor 9. The G and C nucleotides in the interior have at least 50% GC content based on the length of the oligodeoxynucelotide.

By "at least one antigen" as used herein means that a sole antigen can be targeted or more than one antigen can be targeted by the means or carrier or toxin carrier or monoclonal or polyclonal antibodies as described herein such as 2, 3, 4, 5, 6, 7, 8, 9 or 10 antigens. Mixtures of different antigens which can be combined with the means for targeting, carriers or toxin carriers or monoclonal or polyclonal antibodies as described herein are also encompassed by the present invention, as well as fragments of antigens provided that these fragments retain their antibody producing activity.

Further encompassed by the present invention are also mixtures of different carriers or toxin carriers or monoclonal or polyclonal antibodies as described herein combined with different antigens and the administration of these different mixtures of different carriers or toxin carriers or monoclonal or polyclonal antibodies as described herein with different antigens combined to them.

By "consisting essentially of" means that the major elements are present in the composition or vaccine, but also minor ingredients that do not materially affect the composition or vaccine can also be present.

The terms, comprising, consisting of and consisting essentially of can be interchanged throughout the specification.

More specifically the present invention provides a composition comprising means for targeting at least one antigen to dendritic cells and an adjuvant comprising a granulocyte macrophage stimulating factor (GM-CSF) and a CpG oligodeoxynucleotide and/or a CpG-like oligodeoxynucleotide. The means for targeting are combined with the at least one antigen.

These compositions, as described herein, can be used as a drug, preferentially as a vaccine. This vaccine can treat existing disease states and are thus therapeutic vaccines or to prevent development of disease state and thus are prophylactic vaccines. It should be appreciated that prophylactive vaccines generally generate humoral responses while therapeutic vaccines generally generate CD8⁺ T lymphocyte responses. Therapeutic vaccines are of particular interest in the compositions and methods described herein.

The means for targeting include any means such as liposomes combined with antibodies or fragments thereof, which fragments retain their targeting capabilities to dendritic cells, biomaterials such as nanoparticles or microparticles which are combined with antibodies or fragments of antibodies as long as the antibody fragments maintain their targeting capabilities to dendritic cells, toxin vectors combined with at least one antigen or monoclonal or polyclonal antibodies that target dendritic cells, which are combined with at least one antigen, as well as combinations thereof.

In another aspect, the present provides a composition comprising a carrier that targets dendritic cells combined with at least one antigen and an adjuvant comprising a granulocyte macrophage colony stimulating factor (GM-CSF) and a CpG oligodeoxynucleotide and/or a CpG-like oligodeoxynucleotide. In this aspect, the carrier can be any carrier such as liposomes combined with antibodies or fragments thereof that retain their targeting capabilities, biomaterials such as nanoparticles or microparticles which are combined with antibodies or fragments of antibodies as long as the antibody fragments maintain their targeting capabilities, toxin vectors combined with at least one antigen that target dendritic cells and combinations thereof. In another aspect, the monoclonal or polyclonal antibodies that target dendritic cells can be combined with at least one antigen.

Any type of liposomes can be used to entrap the at least one antigen. Any natural or synthetic phospholipids such as phosphoglycerides and sphingolipids can be used to fabricate the liposomes. Natural phospholipids such as phosphatidylchooline (PC), phosphotidylethanolamine (PE) and phosphotidylserine can be used. Synthetic phospholipids that can be used include dioleoylphosphatidylcholine, dioleoylphosphatidylethanolamine, distearoylphosphatidylcholine and distearoylphosphatidylethanolamine. Cholesterol can be incorporated into the liposome depending upon the application. Cholesterol can be incorporated in a concentration varying from 1:1 or even 2:1 molar ratios of cholesterol to PC.

The liposomes can be unilamellar vesicles or multilamellar vesicles. The liposomes can also be cross-linked.

Liposomes of the present invention can be made by methods known in the art using passive loading techniques or active loading techniques. Examples of mechanical dispersion methods include lipid film hydration methods, micro emulsion methods, sonication, French press methods, membrane extrusion methods, dried reconstituted vesicle methods and freeze thawed liposome methods. Solvent dispersion methods include ethanol injection, ether injection, double emulsion vesicles, reverse phase evaporation vesicles and stable plurilamellar vesicles. The use of detergent such as cholate and Triton X^{Ⓡ} 100 and removal of the detergent by dialysis, dilution or column chromatography can also be used for liposome preparation.

The at least one antigen can also be delivered to dendritic cells through nanoparticles. These particles have a size of less than or equal to 100 nm. They can be fabricated from natural materials or derivatives, dendrimers, fullerenes, polymers, silica, albumin, gold, hydrogels and other materials known in the art. Examples of natural materials for fabricating nanoparticles include chitosan, dextran, gelatine, aliginates and starch. The various polymers that can be used in the nanoparticles of the present invention include polylactic acid, poly(cyano) acrylates, polyethylene amine, block copolymers, polycaprolactone and poly(lactic-co-glycolic) acid (PLGA).

The nanoparticles can be coated with various materials such as a dextran coating, an enteric coating, a polymer coating, a gold coating, a polyethyleneglycol (PEG) coating and a carbohydrate coating.

The nanoparticles can be made using different methods such as attrition, pyrolysis, using thermal plasma methods, gas-phase techniques, multiple emulsion-solvent evaporation methods, gas-flow focusing, electrospray, fluidic nanoprecipitation methods, emulsion diffusion-evaporation methods, modified phase inversion/solvent diffusion methods, or sol-gel methods. These methods are described in the literature and known to those skilled in the art.

Microparticles are particles that have a size between 0.1 to 100 µM. They can be fabricated of natural or synthetic polymers using materials similar to those of nanoparticles. Thus, cellulose, starch, lysophosphatidylcholine, poly(lactic acid), phosphorylcholine, poly(DL-lactide-co-glycolide), alginate-spermine, polyamino acids, polyphosphazenes, albumin, dextran, Eudragit S 100, Eudragit L 100, gelatine and 3-(triethoxysilyl)propyl-terminated polydimethylsiloxane are some of the materials that are used to make microparticles. Microparticles of the present invention can also be grafted with other materials. As examples, starch microparticles grafted with polymethyl methacrylate or polyacrylate or silicone-grafted starch microparticles.

Microparticles can also be coated using the same coatings as those described above for nanoparticles; i.e., a dextran coating, an enteric coating, a polymer coating, a gold coating, a Eudragit S 100 coating, a PEG coating and a carbohydrate coating.

In formulating the microparticles several methods can be used such as spray-drying, emulsion/evaporation, double emulsion/evaporation, salting out, solvent displacement/precipitation, cryopreparation, and oil in oil emulsion/solvent evaporation. These and other methods are described in the literature (see, for example, Kendall et al, Eur. J. Pharm, Sci 37, 284-290 (2009)) and are known in the art.

With respect to the means for targeting at least one antigen to dendritic cells or carriers that target to dendritic cells the liposomes, microparticles or nanoparticles are formulated as such that the at least one antigen is on the interior of these carriers. In another aspect the at least one antigen is formulated at the exterior of these carriers. In yet another aspect the at least one antigen is formulated at the interior and exterior of these carriers. Mixtures of different antigens can be formulated in the same manner as described herein.

To encapsulate the at least one antigen on the interior of the liposomes, microparticles or nanoparticles carriers the carriers can be formulated in a media that contains a high concentration of the at least one antigen The non-encapsulated antigen or antigens can be removed from the carriers either by size exclusion chromatography or equilibrium dialysis.

In case it is desired that the at least one antigen is at the exterior of the liposomes, microparticles or nanoparticles carriers the at least one antigen can be combined with the carrier using any means known in the art such as via an electrostatic or hydrophobic interaction or can be covalently linked chemically. The at least one antigen can also be attached to the carrier through a linker. The types of linkers used depend upon the type of carrier, the materials used in the carriers and the at least one antigen to be complexed thereto. Examples of linkers include thiol group linkages, alkyl group linkages, glycol group linkage, a peptide group linkage and alkyl disulphide linkages.

Besides, the at least one antigen attached to the exterior or encapsulated in the interior or both the liposomes, microparticles or nanoparticles have polyclonal or monoclonal antibodies that can target antigens to dendritic cells which are also attached to the exterior of these carriers. These are the means for targeting the at least one antigen to the dendritic cells. Any means of attachment of the antibodies to the liposomes, microparticles, or nanoparticles such as by non-covalent binding, covalent binding, adsorption, via an electrostatic or a hydrophobic interaction can be used.

The antibodies used to target the at least one antigen to the dendritic cells can be polyclonal anibodies, monoclonal antibodies or fragments of polyclonal or monoclonal antibodies as long as the fragments retain their targeting capabilities. The antibodies can also be chimeric antibodies, They also can be mixtures of polyclonal and monoclonal antibodies and fragments thereof. The fragments used in the present invention should retain their capability of binding to dendritic cells. Single chain Fv (scFv), as well as Fab fragments of antibodies that deliver the at least one antigen to dendritic cells can also be used.

In another aspect, diabodies such as those described by Hollinger, P. and Winter, G. in Cancer Immunology, Immunotherapy vol 45 no,3-4 pp.128-130 (1997), bispecific antibodies such as those described in Hollinger et al, PNAS vol. 90 no. 14 pp6444-6448 (1993) and minibodies such as those described by Tramontano et al in Journal of Molecular Recognition vol. 7 no. 1 pp9-24 (1994) can be used.

The antibodies of the present invention can be murine antibodies or human antibodies. The murine antibodies can be subject to "demurinization," if the need arises.

Specific examples of the antibodies that can be used in the present invention to target the compositions to dendritic cells include those antibodies that target the mannose receptor, the Fcγ receptor, the asialoglycoprotein receptor, blood DC antigen 2 (BDCA-2), CD205, NLDC-145, Clec9A, Clec12A, CD11c, CD8, cDC, DCIR-2,FIRE, CIRE, dectin-1, dectin-2, DC-SIGN, L-SIGN, MANR, MMR, MGL, CD23, CD69, CD94, Ly-49 and NKG2. Thus any anti-mannose receptor antibodies, anti-Clec9A antibodies, anti-Clec12A antibodies, anti-CD11c antibodies, anti-CD8 antibodies, anti-cDC antibodies, anti- DCIR-2 antibodies, anti-FIRE antibodies, anti-CIRE antibodies, anti-dectin-1 antibodies, anti-dectin-2 antibodies, anti-DC-SIGN antibodies, anti-L-SIGN antibodies, anti-MANR antibodies, anti-MMR antibodies, MGL anti-antibodies, anti-CD23 antibodies, anti-CD69 antibodies, anti-CD94 antibodies, anti-Ly-49 antibodies and anti- NKG2 antibodies can be used in the compositions and methods of the present invention. Mixtures of various antibodies, as well as fragments of these antibodies can also be used as long as they retain their targeting capabilities.

In a preferred embodiments the antibody is an anti-DEC205 antibody, preferably CD205, NLDC-145, fragments thereof retaining their dendritic cell targeting capabilities, and mixtures thereof.

In one aspect the targeting antibodies targets DEC205 membrane glycoproteins and antibodies that can be used in this aspect are CD205 antibodies and NLDC-145 antibodies and mixtures thereof and fragments thereof as long as the antibody fragments retain their targeting capabilities.

Methods of producing both polyclonal and monoclonal antibodies and fragments thereof are well known in the art. For polyclonal antibodies or fragments thereof an immunogen such as a purified polypeptide, a polypeptide coupled to a carrier such as GTS, keyhole limpet hemanocyanin or any appropriate carrier or a polypeptide incorporated into an immunization vector is mixed with an adjuvant and animals are immunized with the mixture. The animal's response to the immunogen preparation is monitored by taking test bleeds and determining the titer of reactivity to the polypeptide of interest. When appropriate levels of antibody to the immunogen are obtained, blood is collected from the animal and antisera are prepared.

For the preparation of monoclonal antibodies or fragments thereof an animal is immunized with the selected antigen. The antibody forming cells are isolated from the spleen of the animal and fused to tumor cells previously grown in culture. The resulting hybridoma is grown in culture and monoclonal antibodies are then isolated and purified. See, for example Kohler and Milstein Nature 256 (5517) pp. 495 (1975); Riechmann et al Nature 332 (6162) pp.323-327 (1998); Goding Monoclonal Antibodies: Principles and Practice (2nd ed) Academic Press, New York, N.Y. (1986).

In one embodiment, the at least one antigen is directly combined with the dendritic targeting antibody or antibody fragment that retains its dendritic cell targeting capabilities. In this embodiment the at least one antigen is attached to the antibody directly or through a linker. Methods to combine the at least one antigen can be by covalent coupling, as a fusion protein or by electrostatic or hydrophobic interaction or ionic interactions. Examples of linkers that can be used include thiol group linkages, alkyl group linkages, glycol group linkages peptide group linkages and alkyl disulphide group linkages.

Examples of toxin vectors that can be used to target at least one antigen to dendritic cells include Shiga toxin, Shiga-like toxins as described herein, Bacillus anthracis toxin, diphtheria toxin, adenylate cyclase toxin, cholera toxin, pseudomonas exotoxin and the like. The non toxic subunit of these toxins can also be used without any toxic subunit. Methods to combine at least one antigen with these toxins can be by covalent coupling, as a fusion protein or by electrostatic or hydrophobic interaction or ionic interactions.

A composition comprising the B subunit of Shiga toxin or an immunologically functional equivalent thereof, which targets dendritic cells and is combined with at least one antigen and an adjuvant comprising a granulocyte macrophage colony stimulating factor (GM-CSF) and a CpG oligodeoxynucleotide and/or a CpG-like oligodeoxynucleotide is another aspect of the invention.

The sequence of the B subunit of Shiga toxin has been described in Strockbine et al., J. Bacteriol, 170, 1116-22 (1988). The immunologically functional equivalent is one in which the toxin can bind to the Gb3 receptor and/or causes the internalization of an antigen and its presentation to the MHC class I pathway or both MHC class I and class II pathways. Thus, the B subunits of Shiga-like toxins from E. *coli* such as Shiga-like toxin 1, Shiga-like toxin 2 and the Shiga-like toxin 2 variants such as Shiga-like toxin 2c, Shiga-like toxin 2d1, Shiga-like toxin 2d2, Shiga-like toxin 2e, Shiga-like toxin 2f and Shiga-like toxin 2y can all be considered immunologically function equivalents. In an embodiment the B subunits of verotoxin-1 or verotoxin-2 or verotoxin 2c or verotoxin 2v from *E. coli* can be used instead of the B subunit of Shiga toxin in the formulation of the composition of the present invention. It should be noted that verotoxin-1 is an alternative name for Shiga-like toxin 1 and that verotoxin-2 is an alternative name for Shiga-like toxin 2.

The B subunit of Shiga toxin or the immunologically functional equivalent thereof is devoid of toxin activity and hence is not toxic to mammals. Lacking toxicity does not affect the binding of the B subunit of Shiga toxin or immunologically functional equivalents thereof to Gb3 receptors or the entry into the MHC class I or MHC class I or II pathways.

The binding to the Gb3 receptor may be evaluated by methods known in the art such as those described by Tarrago-Trani in Protein Extraction and Purification, 39:pp 170-176 (2004) or Nishikawa et al in Chem Pharm Bull April 54(4): pp.522-7 (2006), which are incorporated herein by reference.

In one embodiment the B subunit of Shiga toxin has the sequence:

COOH-MKKTLLIAASLSFFSASALATPDCVTGKVEYTKYNDDDTFTVKVGDK
ELFTNRWNLQSLLLSAQITGMTVTIKTNACHNGGGFSEVIFRC-NH2 (SEQ ID NO: 4).

The B subunit of Shiga toxin or immunologically equivalent thereof is then combined with at least one antigen.

In another aspect, the B subunit of Shiga toxin or said immunologically equivalent thereof is combined to said at least one antigen through chemical coupling via a Cysteine group or a Z(n)-Cys group wherein Z is an amino acid devoid of a sulfydryl group and n is 0, 1 or a polypeptide.

The at least one antigen that can be used in the compositions, use and methods as described herein include cancer antigens, infectious disease antigens such as bacterial antigens, viral antigens, fungal antigens or parasitic antigens, allergy antigens, autoimmune antigens from warm blooded animals and mixtures of these antigens.

The antigens for cancers can be antigens from testicular cancer, ovarian cancer, pancreatic cancer, melanoma, lung cancer, prostrate cancer, hepatic cancer, breast cancer, rectal cancer, colon cancer, esophageal cancer, gastric cancer, renal cancer, sarcoma, neuroblastoma, Hodgkins and non-Hodgkins lymphoma and leukemia.

Cancer antigens useful for the immunotherapeutic treatment of cancers such as MAGE A1, MAGE A3 or other MAGE antigens for the treatment of melanomas, antigens that are expressed in a large number of tumor types such as melanoma, lung carcinoma, sarcoma and bladder carcinoma such as PRAME, BAGE or GAGE, prostrate specific antigen (PSA), HER-2/neu, KSA, PAP mammaglobin, MUC-1, CEA, WT1, LMP2, HPBV E6, E7, EGFRvIII, ldiotype, p53 nonmutant, NY-ESO-1, NY-ESO-2, NY-ESO-3, NY-ESO-4, NY-ESO-5, NY-ESO-6, NY-ESO-7, NY-ESO-8, PSMA, GD2, MelanA/MART1, Ras mutant, gp100, p53 mutant, Proteinase3(PR1), bcr-abl, Tyronsinase, Survivin, nTERT, Sarcoma translocation breakpoints, EphA2, ML-IAP, AFP, EpCAM, ERG (TMPRSS2ETS fusion), NA17m PAX3, ALK, Androgen receptor, Cyclin B1, Polysialic acid, MTCN, RhoC, TRP-2, GD3, Fucosyl GM1, Mesothelin, PSCA, sLe(a), CYP1B1, PLAdm GM3, BORISm Tn, GloboH, ETV6-AML, NY-BR-1, RGS5, SART3, STn, Carbonic Anhydrase IX, Sperm protein 17, LCK, HMWMAA, AKAP-4, SSX2, XAGE 1, B7H3m Legumain, Tie 2, Page4, VEGFR2, MAD-CT-1, FAP, PDGFR-β. MAD-CT-2 Fos-related antigen 1, some of which are described in Cheever et al, "The Prioritization of Cancer Antigens: A National Institute Pilot Project for the Acceleration of Translational Research," Clinical Cancer Research, 15, pgs, 532305337 (August 31, 2009). In one embodiment HER-2/neu is used as the at least one antigen. Fragments of these cancer antigens can also be used as long as they stimulate antibody production.

The antigens for bacterial infections can be antigens from *Escherichia coli, Salmonella enterica, Neisseria meningitis, Listeria monocytogenes,* bacterial *Meningitis, Chlamydia pneumoniaea, Diptheria, Streptoccoccus pneumoniaea, Streptoccoccus aureus, Streptoccoccus* bacteria, *Heliobacter pylori, Haemophilus influenza* Serotype B, Legionellosis, *Mycoplasma pneumoniae, Pertussis,* scarlet fever, toxic shock syndrome, trachoma, urinary tract infections, anthrax, botulism, cholera, typhus, gonorrhea, impetigo, leprosy, leptospirosis, lyme disease, meliodosis, MRSA infection, nocardosis, pertussis, the plague, *Pneumococcal* pneumonia, psittacosis, Q fever, Rocky Mountain spotted fever, shigellosis, tetanus, tuberculosis, tularemia and typhoid fever and mixtures thereof. Fragments of these bacterial antigens can also be used as long as they stimulate antibody production.

Viral antigens can be antigens from AIDS, such as gag, tat nef, the envelope such as gp120 or gp 160 fragments thereof, *Varicella,* colds, Cytomegalovirus, Colorado tick fever, Dengue fever, Ebola haemorrhagic fever, hand foot and mouth disease, Rotavirus, Coronavirus, hepatitis, herpes simplex, herpes zoster, human papilloma virus, influenza, lassa fever, measles, Marbug hemorrhagic fever, infectious mononucleosis, mumps, poliomyelitis, progressive multifocal leukencephalopathy, rabies, rubella, SARS, variola-zoster virus, viral encephalitis, viral meningitis, viral pneumonia, West Nile disease, influenza A virus, Epstein-bar virus, respiratory syncytial virus, adult T cell leukemia virus, Hepatitis A virus, pox virus and yellow fever and mixtures thereof. Fragments of these viral antigens can also be used as long as they stimulate antibody production.

Fungal antigens that can be used in the present invention include antigens from allergic bronchopulmonary aspergillosis, pulmonary aspergilloma, athlete's foot, basidiobolomycosis, black piedra, blastomycosis, candidiasis, chytridiomycosis, coccidiodomycosis, conidiobolomycosis, covered smut, cryptococcosis, cryptococcus gatti, dermatophytosis, dimorphic fungi, endothrix, entomopathogenic fungus, epizootic lymphangitis, esosphageal candidiasis, exothrix, fungemia, histoplasmosis, massospora cicadina, mycosis, piedraia, pneumocystis pneumonia, sirococcus clavigignenti-juglandacearum, sporotrichosis, thousand cankers disease, tinea, tinea barbae, tinea capitis, tinea corporis, tinea crusis, tinea faciei, tinea incognito, tinea nigra, tinea versicolor, thrush and white nose syndrome and mixtures thereof. Fragments of these fungal antigens can also be used as long as they stimulate antibody production.

Antigens that can be used to treat parasitic or protozoan infectious diseases include antigens from African trypanosomiasis, amebiasis, ascariasis, babesiosis, balatidiasis, chagas disease, clonorchiasis, coccidiosis, cryptosporidiosis, cysticercosis, diphyllobothriasis, dracunculiasis, echinococcosis, enterobiasis, fascioliasis, fasciolopsiasis, filariasis, free-living amebic infection, giardiasis, gnathostomiasis, helminths, hexamitiasis, hymenolepiasis, isosporiasis, leishmaniasis, malaria, metagonimiasis, myiasis, onchocerciasis, pediculosis, plasmonium , scabies, schistosomiasis, taeniasis, toxocariasis, toxoplasmosis, trichinellosis, trichuriasis, trichomoniasis and trypanosomiasis and mixtures thereof. Fragments of these parasitic or protozoan antigens can also be used as long as they stimulate antibody production.

Antigens that can be used to treat allergies include antigens from cigarette smoke allergies, chemical allergies, cloth allergies, cockroach allergies, dust mite allergies, food allergies, gastrointestinal allergies, grass pollen allergies, hay fever, house dust allergies, insect sting or bites allergies, latex allergies, mold allergies, pet allergies, pollen allergies, ragweed allergies and tree pollen allergies and mixtures thereof. Fragments of these allergy antigens can also be used as long as they stimulate antibody production.

Antigens that can be used to treat autoimmune diseases include antigens from achlorhydra autoimmune active chronic hepatitis, Addison's disease, alopecia areata, Amyotrophic Lateral Sclerosis, akylosing spondylitis, anti-GBM Nephritis or anti-TBM nephritis, antiphospholipid syndrome, aplastic anemia, arthritis, asthma, atopic allergy, atopic dermatitis, autoimmune hemolytic anemia, autoimmune hepatitis, autoimmune inner ear disease (AIED), autoimmune lymphoproliferative syndrome (ALPS), Balo disease, Behcet's disease, Berger's disease, bullous pemphigoid, cardiomyopathy, celiac disease, chronic fatigue immune dysfunction syndrome, Churg Strauss syndrome, cicatricial pemphigoid, Cogan's syndrome, cold agglutinin disease, colitis, cranial arteritis, CREST syndrome, Crohn's disease, Cushing's syndrome, Dego's disease, dermatitis, dermatomyositis, Devic disease, diabetes type 1, diabetes type 2, Dressler's syndrome, discoid lupus, essential mixed cryoglobulinemia, eosinophilic facsiitis, epidermolysis bullosa ccquisita, Evan's syndrome, fibromyalgia, fibrosing alveolitis, gastritis, giant cell artertis, glomerulonephritis, Goodpasture's disease, Grave's disease, Guillian-Barre syndrome, Hashimoto's thyroiditis, hemolytic anemia, Henoch-Schonlein purpura, hepatitis, Hughes syndrome, idiopathic adrenal atrophy, idiopathic pulmonary fibrosis, idiopathic thrombocytopenia purpura, inflammatory demylinating polyneuropathy, irritable bowel syndrome, Kawasaki's disease, lichen planus, Lou Gehrig's disease, lupoid hepatitis, Lupus, Lyme disease, Meniere's Disease, mixed connective tissue disease, multiple myeloma, multiple sclerosis, myasthenia gravis, myositis, ocular cicatricial pemphigoid, osteoporosis, pars planitis, pemphigus vulgaris, polyglandular autoimmune syndromes, polymyalgia rheumatica (PMR), polymyositis, primary biliary cirrhosis, primary sclerosing cholangitis, psoriasis, Raynaud's phenomenon, Reiter's syndrome, rheumatic fever, rheumatoid arthritis, sarcoidosis, scleritis, scleroderma, Sjogren's Syndrome, sticky blood syndrome, Still's disease, Stiff Man Syndrome, sydenham horea, systemic Lupus Erythmatosis, Takayasu's arteritis, Temporal arteritis, ulcerative colitis, vitiligo, Wegener's granulomatosis and Wilson's syndrome and mixtures thereof. Fragments of these autoimmune antigens can also be used as long as they stimulate antibody production.

The amount of the at least one antigen used in the compositions and/or methods of the present invention depends on the antigen that is used and thus varies with each different formulation. However the at least one antigen should at least induce an immunoprotective response without adverse side effects. Generally the composition will contain between 0.1 to 1,000 µg of each antigen. In another aspect the composition will contain 0.1 to 500 µg of each antigen. In yet another aspect the composition will contain between 0.1 to 100 µg of each antigen. 0.1 to 50 µg of each antigen can also be used in the composition in yet another aspect.

The composition comprising the means for targeting at least one antigen to dendritic cells or carrier that targets dendritic cells such as liposomes, microparticles and nanoparticles or toxin carriers that target dendritic cells or monoclonal or polyclonal antibodies that target dendritic cells that are combined with at least one antigen by covalent bonding or as a fusion protein or by electrostatic or hydrophobic interaction or ionic interactions. If liposomes, microparticles or nanoparticles are used as the carrier the at least one antigen can be complexed to the outside of these carriers or formulated on the interior by encapsulation. When complexing the at least one antigen on the exterior of liposomes, microparticles or nanoparticles, linkers as described herein can be used. Mixtures of different antigens can be combined with the different carriers described herein.

In one aspect, the composition comprising the B subunit of Shiga toxin or the immunologically equivalent thereof the combining of at least one antigen can also be by covalent bonding or as a fusion protein or by electrostatic or hydrophobic interaction or ionic interactions. The combining can be done directly on the B subunit of Shiga toxin or the immunologically functional equivalent thereof or it can combined through a linker as described herein or a Cysteine group or a Z(n)-Cys group wherein Z is an amino acid devoid of a sulfhydryl group and n is 0, 1 or a polypeptide.

For covalently binding the at least one antigen coupling agents can be used such as cyanogen bromide, cyanuric chloride, 2,2-dichlorobenzidinem p,p'-difluoro-m,m'-dinitrodiphenylsulfone or 2,4-dichloronitobenzene. In an embodiment cyanogen bromide is used to covalently link the at least one antigen.

Fusion proteins as that described in Haicheur et al, Journal of Immunology 165 pgs 3301-3308 (2000), incorporated herein by reference, are also a source of linkage and can be used to couple the antigen to the toxin carrier or the B subunit of Shiga toxin or the immunologically functional equivalent thereof.

Noncovalent bindings include ionic interactions, hydrophobic interactions and binding through hydrogen bonds. Thus with respect to ionic interactions it is well known that negatively charged carboxyl groups on aspartic acid and glutamic acid may be attracted by positively charged free amino groups on lysine and arginine residues.

Once the at least one antigen is combined with the means for targeting at least one antigen to dendritic cells or carrier that is combined with at least one antigen that targets dendritic cells such as liposomes, microparticles and nanoparticles, toxin carriers that are combined with at least one antigen that target dendritic cells or monoclonal or polyclonal antibodies that are combined with to at least one antigen and that target dendritic cells they are then mixed with the adjuvant granulocyte macrophage colony stimulating factor (GM-CSF) and administered to the warm blooded animal in need of such treatment. The amount of GM-CSF that is administered ranges between 2 to 50 µg. In one embodiment 20 µg is administered, in another embodiment 10 µg is administered with the means for targeting at least one antigen to dendritic cells or carrier that is combined with at least one antigen that targets dendritic cells such as liposomes, microparticles and nanoparticles, toxin carriers that are combined with at least one antigen that target dendritic cells, monoclonal or polyclonal antibodies that are combined with at least one antigen that target dendritic cells or the B subunit of Shiga toxin or immunologically equivalent thereof combined with the antigen.

The means for targeting combined with at least one antigen to dendritic cells or carrier that is combined with at least one antigen that targets dendritic cells such as liposomes, microparticles and nanoparticles, toxin carriers that are combined with at least one antigen and target dendritic cells or monoclonal or polyclonal antibodies that are combined with at least one antigen and target dendritic cells or the B subunit of Shiga toxin or its immunologically equivalent thereof combined with at least one antigen is generally administered with the adjuvant GM-CSF at day 0 and the CpG oligodeoxynucleotide or a CpG-like oligodeoxynucleotide can be administered at day 1 in one aspect of the invention.

In another aspect, the means for targeting at least one combined antigen to dendritic cells or carrier that is combined with at least one antigen that targets dendritic cells such as liposomes, microparticles and nanoparticles, toxin carriers that are combined with at least one antigen and target dendritic cells or monoclonal or polyclonal antibodies that are combined with at least one antigen and target dendritic cells or the B subunit of Shiga toxin or its immunologically equivalent thereof combined with at least one antigen can be administered simultaneously on the same day or sequentially on the same or different days or separately on the same or different days with the adjuvant GM-CSF and the CpG oligodeoxynucleotide or a CpG-like oligodeoxynucleotide

Any CpG oligodeoxynucleotide or a CpG-like oligodeoxynucleotide can be used as the second adjuvant. For instance Class A oligodeoxynucleotides having the features of a poly G sequence at the 5' end or at the 3' end or both, an internal palindrome sequence, a partially phosphorothioated modified backbone and GC dinucleotides within the internal palindrome. An example of a Class A oligodeoxynucleotide is 2216 described by Krug et al, European Journal of Immunology, 31 (7) pgs. 2154-63 (2001). The Class B CpG oligodeoxynucleotides feature one or more 6 mer CpG motifs of 5' Pu Py C G Py Pu-3' and are generally 18 to 28 nucleotides in length and have a fully phosphorothioated modified backbone. Oligodeoxynucleotide 2007 falls in Class B and was described by Krieg et al., Nature 374 (6522) Pgs, 546-9 (1995).

Other CpG oligodeoxynucleotides or a CpG-like oligodeoxynucleotide that can be used as the CpG adjuvant include numbers 1758 and 1826 described by Liu et al, Blood, 92 3730-3736 (1998), number 1668, 1720m 2006 and 2041 described by Deng et al, The Journal of Immunology, 167 4616-4626 (2001), number 7909 described by Speiser et al, The Journal of Clinical Investigation volume 115, No, 3, pgs, 739-746 (2005) and numbers 2216, D32 and D19 described by Guzylack-Piriou.

In one embodiment the CpG oligodeoxynucleotide can have the sequence of TCCATGACGTTCCTGACGTT (SEQ ID NO:5)

CpG-like olideoxygonucleotides are also encompassed by the present invention and can be used in place of CpG oligodeoxynucleotides. Alternatively CpG-like and CpG oligonucleotides can be administered together as a mixture.

In one aspect, on day 1 the CpG oligodeoxynucleotide or a CpG-like oligodeoxynucleotide is administered at a dose between 1 to 1000 µg per dose. In another aspect it is administered between 1 to 500 µg per dose. In yet another embodiment it is administered between 1 to 100 µg per dose. In yet another embodiment it is administered between 1 to 50 µg per dose.

In another embodiment the adjuvant GM-CSF and CpG or CpG-like are administered together as adjuvants at day 0.

The warm blooded animal can be administered a booster dose containing the composition comprising the means for targeting at least one antigen to dendritic cells or carrier that is combined with at least one antigen that targets dendritic cells such as liposomes, microparticles and nanoparticles, toxin carriers that are combined with at least one antigen that target dendritic cells or monoclonal or polyclonal antibodies that are combined with at least one antigen that target dendritic cells or the B subunit of Shiga toxin or its immunologically equivalent thereof combined with at least one antigen at least 14 or more days after the initial administration. This booster dose is not administered with the adjuvants.

In another aspect a booster dose containing the B subunit of Shiga toxin or immunologically equivalent thereof combined with the at least one antigen at least 14 or more days after the initial administration. This booster dose is not administered with the adjuvants.

The compositions described herein can be administered mucosally or systemically. If delivered systemically it is generally through transdermal, subcutaneous or intramuscular routes. In one embodiment the composition of the present invention and the adjuvants are administered intramuscularly. In another embodiment the composition of the present invention is injected directly into the tumor.

In one aspect, a composition comprising the means for targeting at least one antigen to dendritic cells or carrier that is combined with at least one antigen that targets dendritic cells such as liposomes, microparticles and nanoparticles, toxin carriers that are combined with at least one antigen that target dendritic cells or monoclonal or polyclonal antibodies that are combined with at least one antigen that target dendritic cells and an adjuvant comprising a granulocyte macrophage colony stimulating factor (GM-CSF) and a CpG oligodeoxynucleotide and/or a CpG-like oligodeoxynucleotide for activating cytotoxic T lymphocytes for treating a disease , in particular for treating cancer, infectious diseases caused by bacterial, viral, fungal, parasitic or protozoan infections, allergies and/or autoimmune diseases, forms part of the invention. In one embodiment, said at least one antigen is an HER-2/neu antigen and said disease is breast cancer.

A composition comprising the B subunit of Shiga toxin or an immunologically functional equivalent thereof which targets dendritic cells and is combined with at least one antigen and an adjuvant comprising a granulocyte macrophage colony stimulating factor (GM-CSF) and a CpG oligodeoxynucleotide and/or a CpG-like oligodeoxynucleotide for activating cytotoxic T lymphocytes for treating a disease , in particular for treating cancer, infectious diseases caused by bacterial, viral, fungal, parasitic or protozoan infections, allergies and/or autoimmune diseases, forms another aspect of the invention. In one embodiment, said at least one antigen is an HER-2/neu antigen and said disease is breast cancer.

Use of a composition comprising the means for targeting at least one antigen to dendritic cells or carrier that is combined with at least one antigen that targets dendritic cells such as liposomes, microparticles and nanoparticles, toxin carriers that are combined with at least one antigen that target dendritic cells or monoclonal or polyclonal antibodies that are combined with at least one antigen that target dendritic cells and an adjuvant comprising a granulocyte macrophage colony stimulating factor (GM-CSF) and a CpG oligodeoxynucleotide and/or a CpG-like oligodeoxynucleotide for activating cytotoxic T lymphocytes and/or for treating a disease in particular for treating cancer, infectious diseases caused by bacterial, viral, fungal, parasitic or protozoan infections, allergies and/or autoimmune diseases, is another aspect of the present invention. In one embodiment, said at least one antigen is an HER-2/neu antigen and said disease is breast cancer.

Use of a composition comprising the B subunit of Shiga toxin or an immunologically functional equivalent thereof which targets dendritic cells and is combined with at least one antigen and an adjuvant comprising a granulocyte macrophage colony stimulating factor (GM-CSF) and a CpG oligodeoxynucleotide and/or a CpG-like oligodeoxynucleotide for activating cytotoxic T lymphocytes and/or treating a disease, in particular for treating cancer, infectious diseases caused by bacterial, viral, fungal, parasitic or protozoan infections, allergies and/or autoimmune diseases, is yet another aspect of the present invention. In one embodiment, said at least one antigen is an HER-2/neu antigen and said disease is breast cancer.

Use of a composition comprising the means for targeting at least one antigen to dendritic cells or carrier that is combined with at least one antigen that targets dendritic cells such as liposomes, microparticles and nanoparticles, toxin carriers that are combined with at least one antigen that target dendritic cells or monoclonal or polyclonal antibodies that are combined with at least one antigen that target dendritic cells and an adjuvant comprising a granulocyte macrophage colony stimulating factor (GM-CSF) and a CpG oligodeoxynucleotide and/or a CpG-like oligodeoxynucleotide for the manufacture of a medicament for treating cancer, infectious diseases caused by bacterial, viral, fungal, parasitic or protozoan infections, allergies and/or autoimmune diseases in warm blooded animals is another aspect of the present invention.

Use of a composition comprising the B subunit of Shiga toxin or an immunologically functional equivalent thereof which targets dendritic cells and is combined with at least one antigen and an adjuvant comprising a granulocyte macrophage colony stimulating factor (GM-CSF) and a CpG oligodeoxynucleotide and/or a CpG-like oligodeoxynucleotide for the manufacture of a medicament for treating cancer, infectious diseases caused by bacterial, viral, fungal, parasitic or protozoan infections, allergies and/or autoimmune diseases in warm blooded animals is yet another aspect of the present invention.

The present invention also relates to the use of a composition comprising the means for targeting at least one antigen to dendritic cells or carrier that is combined with at least one antigen that targets dendritic cells such as liposomes, microparticles and nanoparticles, toxin carriers that are combined with at least one antigen that target dendritic cells or monoclonal or polyclonal antibodies that are combined with at least one antigen that target dendritic cells, and an adjuvant comprising a granulocyte macrophage colony stimulating factor (GM-CSF) and a CpG oligodeoxynucleotide and/or a CpG-like oligodeoxynucleotide for the manufacture of a medicament to treat breast cancer. In one embodiment, said at least one antigen is an HER-2/neu antigen.

Use of a composition comprising the B subunit of Shiga toxin or an immunologically functional equivalent thereof which targets dendritic cells and is combined with an HER-2/neu antigen and an adjuvant comprising a (GM-CSF) and a CpG oligodeoxynucleotide or a CpG-like oligodeoxynucleotide for the manufacture of a medicament to treat breast cancer is yet another embodiment of the present invention.

The immunologically functional equivalents are described above for the composition and apply here with respect to the use of this composition. They include any toxin that can bind to the Gb3 receptor and/or causes the internalization of an antigen and its presentation to the MHC class I pathway or both MHC class I and class II pathways. Thus, the B subunits of Shiga-like toxins from E. *coli* such as Shiga-like toxin 1, Shiga-like toxin 2 and the Shiga-like toxin 2 variants such as Shiga-like toxin 2c, Shiga-like toxin 2d1, Shiga-like toxin 2d2, Shiga-like toxin 2e, Shiga-like toxin 2f and Shiga-like toxin 2y can all be considered immunologically function equivalents In an embodiment the B subunits of verotoxin-1 or verotoxin-2 or verotoxin 2c or verotoxin 2v from E. *coli* can be used instead of the B subunit of Shiga toxin in the formulation of the composition of the present invention.

The B subunit of Shiga toxin or the immunologically functional equivalent thereof which targets dendritic cells is combined in the same manner as set forth above concerning the compositions; i.e., by covalent bonding or by electrostatic or hydrophobic interaction or as a fusion protein.

The same antigens set forth above with respect to the composition can be used in the use aspects of the invention such as cancer antigens and the HER-2/neu antigen.

Likewise the same CpG oligodeoxynucleotides and/or a CpG-like oligodeoxynucleotide as set forth above in the compositions can be used in the use aspects of the invention.

A method for activating cytotoxic T lymphocytes said method comprising administering to a warm blooded animal in need of such activation a composition comprising the means for targeting at least one antigen to dendritic cells or carrier that is combined with at least one antigen that targets dendritic cells such as liposomes, microparticles and nanoparticles, toxin carriers that are combined with at least one antigen that target dendritic cells or monoclonal or polyclonal antibodies that are combined with at least one antigen that target dendritic cells and an adjuvant comprising a granulocyte macrophage colony stimulating factor (GM-CSF) and a CpG oligodeoxynucleotide and/or a CpG-like oligodeoxynucleotide is yet another aspect of the invention.

A method for activating cytotoxic T lymphocytes said method comprising administering to a warm blooded animal in need of such activation a composition comprising the B subunit of Shiga toxin or an immunologically functional thereof which targets dendritic cells and combined with at least one antigen and an adjuvant comprising a granulocyte macrophage colony stimulating factor (GM-CSF) and a CpG oligodeoxynucleotide and/or a CpG-like oligodeoxynucleotide is yet another aspect of the invention.

A method of treating cancers, infectious diseases caused by bacterial, viral, fungal, parasitic or protozoan infections, allergies and autoimmune diseases said method comprising administering to a warm blooded animal in need of such treatment a composition comprising the means for targeting at least one antigen to dendritic cells or carrier that is combined with at least one antigen that targets dendritic cells such as liposomes, microparticles and nanoparticles, toxin carriers that are combined with at least one antigen that target dendritic cells or monoclonal or polyclonal antibodies that are combined with at least one antigen that target dendritic cells and an adjuvant comprising a granulocyte macrophage colony stimulating factor (GM-CSF) and a CpG oligodeoxynucleotide and/or a CpG-like oligodeoxynucleotide is still another aspect of the present invention.

In yet another aspect a method of treating cancers, infectious diseases caused by bacterial, viral, fungal, parasitic or protozoan infections, allergies and autoimmune diseases said method comprising administering to a warm blooded animal in need of such treatment a composition comprising the B subunit of Shiga toxin or an immunologically functional thereof which targets dendritic cells and is combined with at least one antigen and an adjuvant comprising a granulocyte macrophage colony stimulating factor (GM-CSF) and a CpG oligodeoxynucleotide and/or a CpG-like oligodeoxynucleotide is provided.

A method of treating breast cancer said method comprising administering to a warm blooded animal in need of such treatment a composition comprising the means for targeting a HER2/neu antigen to dendritic cells or carrier that is combined with a HER2/neu antigen that targets dendritic cells such as liposomes, microparticles and nanoparticles, toxin carriers that are combined with a HER2/neu antigen that target dendritic cells or monoclonal or polyclonal antibodies that are combined with a HER2/neu antigen that target dendritic cellsand an adjuvant comprising a granulocyte macrophage colony stimulating factor (GM-CSF) and a CpG oligodeoxynucleotide and/or a CpG-like oligodeoxynucleotide is provided.

A method of treating breast cancer said method comprising administering to a mammal in need of such treatment a composition comprising the B subunit of Shiga toxin or an immunologically functional thereof which targets dendritic cells and is combined with a HER-2/neu antigen and an adjuvant comprising a granulocyte macrophage colony stimulating factor (GM-CSF) and a CpG oligodeoxynucleotide and/or a CpG-like oligodeoxynucleotide.

A kit for vaccination comprising at least one of the compositions of the present invention as described herein and an adjuvant comprising a granulocyte macrophage colony stimulating factor (GM-CSF) and a CpG oligodeoxynucleotide and/or a CpG-like oligodeoxynucleotide and at least one pharmaceutically acceptable vehicle is yet another embodiment of the invention.

The pharmaceutically acceptable vehicles include sterile water, saline or buffered solutions.

The vaccine or the kit for vaccination as described herein is suitable for simultaneous, sequential or separate administration to a warm blooded animal.

A combination of products (also designated as a kit of parts) comprising a composition comprising the means for targeting to dendritic cells combined with the at least one antigen or a carrier which is combined with the at least one antigen that targets dendritic cells such as liposomes, microparticles and nanoparticles, toxin carriers that are combined with at least one antigen that target dendritic cells or monoclonal or polyclonal antibodies combined with at least one antigen that target dendritic cells, or a B subunit of Shiga toxin or an immunologically functional thereof which targets dendritic cells and is combined with at least one antigen, and an adjuvant comprising a granulocyte macrophage colony stimulating factor (GM-CSF) and a CpG oligodeoxynucleotide and/or a CpG-like oligodeoxynucleotide for administration simultaneously, sequentially or separately is still yet another aspect of the present invention.

A number of embodiments and/or aspects of the invention have been described. Nevertheless it will be understood that various modifications may be made without departing from the spirit and scope of the invention.

### Examples

### Example 1 -Coupling of the B subunit of Shiga toxin to an Antigen

### 1A- Recombinant Coupling of the B subunit of Shiga toxin to MAGE

The process for coupling the B subunit of Shiga toxin (STxB) to a MAGE antigen is described in U.S. Patent 6,613,882, incorporated herein by reference. More specifically, the plasmid used was the pSU108 plasmid described by Su et al, Infect lmmun, 60, pgs. 33-45 (1992).

The PCR primers that were used were:

5'-CTAGCTCTGAAAAGGATGAACTTTGAGAATTCTGACTCAGAATAGCTC-
3' (SEQ ID NO.6)

5’-CTTTTCAGAGCTAGTAGAATTAGGATGATAGCGGCCGCTACGAAAAATAACT
TCGC-3’ (SEQ ID NO: 7)

The primers used were specific primers from the ShigaAtpE (5') vector and had the following sequences:
primer ShigaAtpE: 5'-CACTACTACGTTTTAAC-3" (SEQ ID NO: 8)
primer Shiga-fd: 5'-CGGCGCAACTATCGG-3'(SEQ ID NO: 9)

These primers produced fragments which were cloned at the restriction sites *Sph*l and Sa/l of the SU108 plasmid.

Adaptor fragments containing the glycosylation site and the KDEL sequence composed of the oligonucleotide sulfate 1: (5'-phosphorylated; 5'-GGCCGCCATCCTAATTCTACTTCT-3') (SEQ ID NO: 10) and sulfate 2 (5'-CTCAGAAGTAGAATTAGGATGGC-3') (SEQ ID NO: 11) (or sulfate 3 (5'-GAGTCTGAAAAAGATGAACTTTGATGAG-3)' (SEQ ID NO: 12) were ligated overnight at 16°C.

The resulting fragments were cloned at the *Not*l and *Eco*RI restriction sites of pSU108 and containing the cDNA coding for B-Glyc-KDEL.

The recombinant fragments were also purified using the technique described by Su et al, *supra.* In brief, *E.coli* cells containing recombinant expression plasmids obtained from pSU108 were cultured overnight at 30° C. The culture was then diluted 5 times in LB supplemented with 50 mg/ml of ampicillin at 50°C. After incubation for 4 hours at 42°C, the cells were thoroughly washed with 10 mM Tris/HCL, pH 8, incubated for 10 minutes in 10 mM Tris/HCL, pH 8, 25% sucrose 1 mM EDTA and finally rapidly resuspended in a water-ice mixture containing 1 mM of PMSF and a protease inhibitor mixture (leupeptin, chymostatin, pepstatin, antipain and aprotinin). The final step led to the rupture of the periplasm. After clarification, the supernatant was charged onto a QFF column (Pharmacia) and eluted with a linear gradient of NaCl in 20 mM Tris/HCL, pH 7.5. Depending on the construction, the B fragment was eluted between 120 mM and 400 mM. The fractions containing the B fragment were then dialyzed against 20 mM of Tris/HCL, pH 7.5 and recharged onto a monoQ column (Pharmacia) and eluted in the same manner as before. The resulting proteins, estimated to have a degree of purity of 95% using polyacrylamide-SDS gel electrophoresis were then stored at -80° C until use.

### 1B- Chemical coupling of the B subunit of Shiga toxin

A plasmid expressing STxB was first prepared according to U.S. Patent 7,632,514, incorporated herein by reference. More specifically the plasmid pSU108 described by Su et al, *supra* was modified to introduce the Cysteine codon tgt at the 3' end of the B-fragment cDNA using SEQ ID NO: 10 and SEQ ID NO: 11 were used with plasmid specific primers ShigaAtpE and Shiga-fd and primers A and B to produce DNA fragments, which, in a second PCR with primers Shiga AtpE and Shiga-fd yield fragments that were cloned into the *Sph*l and Sa/l restriction sites of pSU108. Sequences derived by PCR were verified by dideoxy sequencing.
Primer A: 5'-AGCGAAGTTATTTTTCGTTGTTGACTCAGAATAGCTC-3' (SEQ ID NO: 13)
Primer B: 5'-GAGCTATTCTGAGTCAACACGAAAAATAACTTC-3' (SEQ ID NO: 14)

The periplasmic extract was obtained by inoculating 125 ml of LB/Amp with 125 µl of an overnight culture grown at 30°C and further grown overnight at 30°C. The culture was then transferred into 375 ml of LB/Amp at 50°C and incubated at 42°C for 4 hours. The culture was then centrifuged to pellet the cells, which were then washed three (3) times with 10 mM Tris/HCL, pH 8.0. The cells were then resuspended in 200 ml of 25% sucrose, 1 mM EDTA, 10 mM TRIS/HCL, and pH 8.0 and incubated at room temperature for 10 minutes. The cells were then further centrifuged to pellet the cells and resuspended in 200 ml of ice cold water containing a protease inhibitor cocktail. The resuspended cells were incubated on ice for 10 minutes, centrifuged and the supernatant was collected. 20 mM Tris/HCL. PH 8.0 was added.

The periplasmic extract was loaded on a QFF anion exchanges column (Pharmacia) and eluted at 230 mM NaCl. StxB-Cys containing fractions were pooled, diluted 4-fold and loaded on a Mono Q anion exchange column (Pharmacia), followed by elution at 230 mM NaCl. After concentration with microconcentration devices from PallFiltron, the pooled fractions were passed through a Sephadex 75 gel filtration column. Purity was above 95%.

The B-fragments of STxB-Cys were essentially monomeric. However, some constructions in which the cysteine was added at more than 2 amino acids from the natural C-terminus of the B-fragment in which neighboring fragments within a pentamer are engaged in disulfide bonds.

Three different carriers were made which were the following:
STxB-Cys in which the Cys has been added directly the C-terminus of the B subunit. This protein eluted as a monomer from the purification columns.

STxB-Z₂-Cys is a carrier with a short spacer of 2 amino acids resulting from the cloning cassette between the C-terminus of the wild type B fragment and the Cys. The majority of the protein eluted as dimmers from the purification columns. These can be separated under reducing conditions, indicating the formation of disulfide bonds between the monomers in the pentameric B-subunit complex.

STxB-Glyc-Cys-KDEL is a carrier in which the Cys is located between a glycosylation cassette being 9 amino acids long and a C-terminal KDEL peptide. The majority of the protein eluted as dimmers from the purification columns. These can be separated under reducing conditions, indicating the formation of disulfide bonds between the monomers in the pentameric B-subunit complex.

Three antigenic peptides were coupled to STxB, which were Pep1, which is a synthetic peptide of 16 amino acids carrying the SL8 antigenic peptide derived from chicken ovalbumin, Pep 2, which is a synthetic peptide of 24 amino acids carrying the SL8 antigenic peptide derived from chicken ovalbumin and a His-gag at its C-terminus and SL8 the antigenic peptide from ovalbumin that can directly exchange with peptides on MHC class I complexes at the plasma membrane of antigen presenting cells.

Two types of conditions were used for coupling the antigenic peptides to the STxB; reducing conditions and non-reducing conditions. Under reducing conditions the fusion proteins were treated with DTT overnight, then the activated peptides, carrying a bromide acetate group at their N-terminal was added in excess. Conditions used for the first coupling experiments using fusion proteins will mostly dimerize monomers (proteins STxBZ₂-Cys and STxB-Glyc-Cys-KDEL). Under non-reducing conditions the fusion proteins were directly reacted with the peptides that were activated with bromide acetate at their N-terminal.

The optimal conditions for the coupling that were used were as follows:
The STxB-Cys was dialyzed against 20 mM Borate buffer, pH 9.0, 150 mM NaCl. After dialysis the STxB-Cys was concentrated to 1 mg/ml. The N-terminally activated peptides were activated with bromoacetate anhydride. The N-terminally activated peptides were dissolved in 12 mM DMSO. The activated peptides were then diluted to 0.2 mM in protein solution and incubated for 12 hours at room temperature. The coupled STxB-activated peptides were then dialyzed against PBS.

### 1C-Chemical coupling of Ova₂₅₇₋₂₆₄, IBC002 and HER2/Neu peptides

### 1.1-Synthesis of StxB

The STxB was prepared from the plasmid pSU108 described by Su et al, *supra* as set forth above but without the Cys at the C-terminus.

### 1.2-Synthesis of the Ova₂₅₇₋₂₆₄, and HER-2/neu

Ova₂₅₇₋₂₆₄, IBC002(SEQ ID NO: 1) and HER-2/neu were chemically synthesized at a scale of 100 µmol of Fmoc-Leu-Wang resin, IRIS Biotech Tentage; 0.25 mmol/g, according to the Fmoc/tBu strategy from the C-terminus to the N-terminus.

The successive coupling of amino acids on the resin was performed on an automatic peptide synthesizer of the Liberty CEM type, using microwaves.

### 1.3-Introduction of a bromoacetyl group at the N-terminus

The peptides were then treated manually.

The Fmoc protection was eliminated by incubation with a mix of DMF:piperidine (80:20), for 5 minutes and then 15 minutes under agitation. The resin was then washed 4 times with DMF.

20 equivalents of bromoacetic acid was activated as an anhydride by 20 minutes of agitation with 10 equivalents of diisopropylcarbodiimide (DIC) in 4 ml dichloromethane that was dried prior to use on a gel filtration column. After concentration in a rotary evaporator, the bromoactic anhydride was solubilized in 4 ml DMF and added to the resin. After agitation for 30 minutes, the resin was washed three times for 2 minutes with DMF, three times 2 minutes with DCM and finally twice with ether before being dried with a membrane vacuum pump.

### 1.4-Cleavage, deprotection and purification

The 2 resins were treated for 1 hour under agitation with a cleavage solution containing 8 ml of TFA and 400 µl of anisole. After precipitation and two washing steps on ice with a 1:1 mixture of ether:heptane, the peptides were taken up in a solution of 5% acetic acid before being injected for preparative HPLC.

The fractions that contained the peptides were pooled, frozen and lyophilized.

### 2. Coupling of the 2 bromoacetylated peptides to STxB/Cys

### 2.1 Conditions chosen to form the thioether link

The cysteine that is present in the STxB-Cys can specifically react with the bromoacetyl function that is present on the peptides to form a covalent thioether link. The following conditions were tested: 4 µl of STxB/Cys at 5 mg/ml in PBS and 1, 2 or 9 equivalents of peptide in 11 µl of PBS. After incubation for 1 night under agitation at room temperature, the solutions were analyzed by MALDI mass spectroscopy. On the MALDI spectra, one observed the quasi- quantitative formation of the conjugate.

The coupling reaction of these two peptide being quasi total in all conditions, it was decided to perform the coupling reaction with an excess of 1.5 equivalents to have a maximal consumption of STxB-Cys without having to remove too much peptide during purification.

### 2.2 Coupling of peptides to 200 µg of STxB-Cys

The following conditions were used: 40 µl of STxB at 5 mg/ml PBS, 15 µl of peptide at 2.56 mM in water and 95 µl of PBS. After incubation for 1 night at room temperature, the reaction was shown to be terminated using LC/MC.

### 2.3 Coupling of the 2 peptides on 5 mg of STxB-Cys

The scale was increase based on the result in 2.2: 220 µl of STxB at 5 mg/ml PBS, 75 µl of peptide at 2.56 mM in water and 475 µl of PBS. These products were purified by gel filtration chromatography.

### Example 2-Comparison of the Elispot Assay after vaccination of mice with STxB-OVA or OVA alone in association with GM-CSF/CpG

Four (4) mice in each group (n=4 and three experiments) were immunized with 20 µg of the B subunit of Shiga toxin (STxB) coupled to ovalbumin (OVA) or OVA alone mixed with 20 µg GM-CSF at day 0. 50 µg of CpG TCCATGACGTTCCTGACGTT (SEQ ID NO: 5) was administered to the mice the day after. On day 14 the mice each of the groups were administered only (STxB) coupled to ovalbumin (OVA) or OVA alone without adjuvant. At day 21 splenocytes were taken from the mice and mononuclear cells were isolated from the splenocytes. An Elispot assay was then performed.

More specifically the Elispot assay was performed as follows. The coating antibody of LT-CD8 anti-OVA was diluted to 15 µg/ml in sterile PBS. 100 µl was added to each 96-well Millipore plate and incubated overnight at 4°C. The plate was washed four times and the non specific binding sites were blocked with blocking buffer (RPMI with 10% FCS).

At day 21 the spleen was removed and mononuclear cells were isolated from splenocytes obtained from the mice by using Ficoll-hypaque. The cells were washed and the viable cells were counted. The cells were resuspended in tissue culture medium at RPMI at a concentration of 4 x 10⁶ cells/ml. 100 µl of cell suspension was added per well.

The stimulus concentration was adjusted to 2X (1-10 µg/ml) and 100 µl of the stimulus was added to each well. The cells were incubated for 40 hr at 37°C in a humid 5% CO₂ incubator.

The Elispot was developed by removing the cells from the wells and washing them 6 times. The cells were removed by washing twice in distilled water and 4 times in washing buffer. The biotinylated mAb (anti-OVA₂₅₇₋₂₆₄) was diluted in blocking buffer to 1 µg/ml and 100 µl was added to each well. The plates were incubated at room temperature for 1-2 hours. Streptavidin alkaline phosphatase was diluted 1:1000 in blocking buffer and 100 µl was added to each well. The plates were then incubated at room temperature for 1-2 hours. After the wells were washed 100 µl of substrate was added and the plates were incubated until dark spots appeared, The color development was stopped by washing in tap water and the plates were left to dry.

The number of spots was counted using an automated machine.

The results are shown in Figure 1. As shown in this Figure more CTL induction was achieved when STxB-OVA was administered with the adjuvants GM-CSF and CpG than OVA alone administered with the same adjuvant.

### Example 3- Comparative Analysis of anti-OVA LT-CD8 induction after vaccination with STxB-OVA or OVA alone using various Adjuvants

Mice (n=4 and two experiments) were immunized with the B subunit of Shiga toxin coupled to OVA or OVA alone using various adjuvants. STxB-OVA was administered with the combination of:
20µg GM-CSF/IFA (vol/vol of IFA), 10µg GM-CSF/IFA, 20µg GM-CSF and 50µg CpG and 1µg of αGalCer. Ova was administered with 20µg GMCSF/IFA and 20µg GM-CSF/50 µg CpG. The secondary adjuvant of CpG was administered the next day. After 14 days the antigen was again administered without the adjuvant. At day 21 splenocytes were taken from the mice and mononuclear cells were purified by Ficoll. The percent of CTL recognizing the tetramer K^{b}-OVA₂₅₇₋₂₆₄/LT-CD8 peptide was measured. As a control K^{b}-VSV (Vascular Stomatitis Virus) was used and the background noise obtained from the control was deducted from the percentage shown.

The results are shown in Figure 2 as the mean result obtained plus or minus standard deviation. As shown in Figure 2 a strong induction of anti-OVA₂₅₇₋₂₆₄ LT-CD8 occurred when the mice were vaccinated with GM-CSF/CpG as compared with other adjuvants, OVA₂₅₇₋₂₆₄ alone had little induction.

### Example 4-Synergy between GM-CSF and CpG as an adjuvant in association with the B subunit of Shiga toxin shown by Tetramer Analysis

Mice (n=4 and two experiments) were immunized with 20 µg of the B subunit of Shiga toxin coupled to a Her2/neu peptide STxB-Her2/neu) using either 50 µg CpG or 20 µg of GM-CSF or a combination of both 50 µg CpG and 20 µg of GM-CSF . The CpG was administered 24 hours after the STxB-HER-2/neu in cases where the combination with GMCSF was used. A second immunization with STxB-HER2/neu alone was carried out at day 14. The splenocytes were removed at day 21 and the LT-CD8 was purified by Miltenyi column. The percent of CTL recognizing the HER2/neu HLA-A2 restricted peptide per tetramer was measured by cytometry. An irrelevant tetramer was used in each experiment to assure the specificity of the reaction against HER2/neu. The background noise obtained from the irrelevant polymer was deducted from the percentage shown.

### The results are shown in Figure 3.

### Example 5- Synergy between GM-CSF and CpG as an adjuvant in association with the B subunit of Shiga toxin shown by the Elispot Assay

Tg HLA-A2 (Pascolo S.J. J. Exp. Med. 1997) mice were immunized twice with 20 µg of the B subunit of Shiga toxin (StxB) coupled to the peptide IBC002, which had the sequence (RRARKIFGSLAFL) (SEQ ID NO:1). Corresponding to the HER2/neu peptide (KIFGSLAFL) (SEQ ID NO: 2) restricted by HLA-A2 and a flanking sequence (RRAR)(SEQ ID NO: 3), which facilitated the processing or with the peptide IBC002 that is non vectorized or with the natural HER2/neu peptide without the flanking sequence in association with 20 µg GM-CSF. The next day 50 µg of CpG is administered. A second immunization with the antigens without adjuvant was performed at day 14. The splenocytes from the mice were then removed and mononuclear cells were purified with Ficoll. Some of the purified splenocytes were sensitized with the natural HER2/neu peptide. The results are given in Figure 4.

These results, shown in Figures 3 and 4, demonstrate the cytotoxic T-lymphocyte (CTL) induction when STxB was administered with an antigen HER2/neu and the adjuvants GM-CSF and CpG. This effect was not observed when the HER2/neu peptide was administered alone. Strongly suggesting that there is synergy between the GM-CSF and CpG adjuvants when administered with an STxB vector coupled to an antigen.

### Example 6-Presentation to Dendritic Cells

OVA is coupled to the B subunit of Shiga toxin following the above procedure in Example 1 C. Basically OVA linked to STxB-Cys by chemically coupling. OVA₂₅₇₋₂₆₄ is first activated via amino groups on lysine side chains using the heterobifunctional crosslinker of MBS (Pierce, Rockford, II). Activated OVA is then reacted with STxB-Cys and the reaction product is purified by gel filtration.

The D1 dendritic cell line is cultured in IMDM (Sigma) supplemented with 10% heat activated FCS, 2 mM glutamine (Sigma), 5 mM sodium pyruvate and 50 µM 2-mercaptoethanol with 30% conditioned medium from granulocyte macrophage colony stimulating factor-producing NIH-3T3 (R1 medium) as described by Winzler et al J. Exp, Med: 185:317 (1997). B3Z is a CD8+ T cell hybridoma specific for the OVA ₂₅₇₋₂₆₄ peptide in the context of K^{b}, which carries a *LacZ* construct driven by NF-AT elements from the IL-2 promoter.

For OVA-derived SL8 K^{b}-restricted peptide presentation, 10⁵ dendritic cells are first pulsed with antigen for 5 hours and washed twice and cocultured overnight using B3Z hybridoma cells (2 x 10⁵ cells/well). A colorimetric assay with ONPG(o-nitrophenyl β-D-galactopyranoside as substrate is used to detect β-galactosidase activity in B3Z lysates.

D1 dendritic cells sensitized with full-size OVA chemically coupled to STxB-Cys are able to present the immunodominant OVA-derived SL8 peptide to B3Z an anti-SL8-specific CD8- T cell hybridoma. As low as 5 nM STxB-Cys-OVA is sufficient to sensitize the D1 cell line for SL8 presentation. In contrast those cell lines that are incubated with OVA alone without STxB-Cys failed to allow presentation of the SL8 peptide.

While the invention has been described in terms of various preferred embodiments, the skilled artisan will appreciate that various modifications, substitutions, omissions and changes may be made without departing from the scope thereof. Accordingly, it is intended that the scope of the present invention be limited by the scope of the claims, including equivalents thereof.

## Claims

1. A composition, preferentially a pharmaceutical composition, comprising means for targeting at least one antigen to dendritic cells, said at least one antigen being combined with said means for targeting, and an adjuvant comprising a granulocyte macrophage colony stimulating factor (GM-CSF) and a CpG oligodeoxy nucleotide and/or a CpG-like oligodeoxynucleotide.

2. The composition according to Claim 1, wherein said means for targeting is a carrier that targets dendritic cells, said carrier being liposomes complexed with antibodies, microparticles complexed with antibodies, nanoparticles complexed with antibodies, toxin carriers or monoclonal or polyclonal antibodies.

3. The composition according to Claim 1 or Claim 2, wherein said at least one antigen is combined with said means for targeting dendritic cells or with said carrier that targets dendritic cells via covalent bonding, or by electrostatic interaction or by hydrophobic interaction or a fusion protein or a chimeric protein.

4. The composition according to any one of Claims 1 to 3, wherein said at least one antigen is an antigen from cancer, an antigen from infectious diseases such as a bacterial antigen, a viral antigen, a fungal antigen a parasitic or protozoan antigen, an allergy antigen or an autoimmune antigen or mixtures thereof.

5. The composition according to any one of Claims 1 to 4, wherein said at least one antigen is a HER-2/neu antigen.

6. The composition according to any one of Claim 1 to 5, wherein said CpG oligodeoxynucleotide has the sequence of TCCATGACGTTCCTGACGTT (SEQ ID NO: 5).

7. The composition according to any one of Claims 1 to 6, wherein said means for targeting said at least one antigen to dendritic cells is a toxin carrier which is a B subunit of Shiga toxin, such as the B subunit of Shiga toxin of SEQ ID N0:4, or an immunologically functional equivalent thereof, said immunologically functional equivalent being preferentially Shiga-like toxin 1, Shiga-like toxin 2, Shiga-like toxin 2c, Shiga-like toxin 2d1, Shiga-like toxin 2d2, Shiga-like toxin 2e, Shiga-like toxin 2f, Shiga-like toxin 2y, verotoxin-1, verotoxin-2, verotoxin 2c or verotoxin 2v.

8. The composition according to claim 7, wherein said B subunit of Shiga toxin or said immunologically equivalent thereof is combined to said at least one antigen through chemical coupling via a Cysteine group or a Z(n)-Cys group wherein Z is an amino acid devoid of a sulfydryl group and n is 0, 1 or a polypeptide.

9. The composition according to any one of Claims 1 to 6, wherein said means for targeting the at least one antigen to dendritic cells is an anti-DEC205 antibody, preferentially CD205, NLDC-145, fragments thereof retaining their dendritic cell targeting capabilities, and mixtures thereof.

10. The composition according to any one of Claims 1 to 9, wherein said at least one antigen is a mixture of different antigens combined with the same or different means or carrier.

11. The composition according to any one of the above claims as a drug, preferentially as a vaccine.

12. Use of a composition according to any one of Claims 1 to 10, for treating cancers, infectious diseases caused by bacteria, viruses, fungus, parasite or protozoans, allergies and/or autoimmune diseases.

13. The use according to Claim 12, for treating breast cancer.

14. A kit for vaccination comprising the composition of any one of Claims 1 to 10 and a pharmaceutically acceptable vehicle.

15. The composition according to any one of Claims 1 to 10, which is a vaccine or the kit for vaccination according to claim 14 suitable for simultaneous, sequential or separate administration to a warm blooded animal.


SEQUENCE LISTING

<110>  INSTITUT CURIE
       CENTRE NATIONAL DE LA RECHERCHE SCIENTIFIQUE
       ASSISTANCE PUBLIQUE-HOPITAUX DE PARIS
       INSERM
       UNIVERSITE RENE DESCARTES - PARIS 5

<120>  COMPOSITIONS HAVING MEANS FOR TARGETING AT LEAST ONE ANTIGEN TO
       DENDRITIC CELLS

<130>  B09319-JAZ/SDC(SDU)

<140>  EPXXXXXXXX.X
<141>  2011-07-22

<160>  14

<170>  PatentIn version 3.3

<210>  1
<211>  13
<212>  PRT
<213>  artificial

<220>
<223>  peptide IBC002

<400>  1

Arg Arg Ala Arg Lys Ile Phe Gly Ser Leu Ala Phe Leu
1               5                   10


<210>  2
<211>  9
<212>  PRT
<213>  artificial

<220>
<223>  HER-2/neu peptide

<400>  2

Lys Ile Phe Gly Ser Leu Ala Phe Leu
1               5


<210>  3
<211>  4
<212>  PRT
<213>  artificial

<220>
<223>  sequence

<400>  3

Arg Arg Ala Arg
1


<210>  4
<211>  94
<212>  PRT

<213>  artificial

<220>
<223>  B subunit of Shiga toxin has the sequence

<400>  4

Cys His Met Lys Lys Thr Leu Leu Ile Ala Ala Ser Leu Ser Phe Phe
1               5                   10                  15

Ser Ala Ser Ala Leu Ala Thr Pro Asp Cys Val Thr Gly Lys Val Glu
            20                  25                  30

Tyr Thr Lys Tyr Asn Asp Asp Asp Thr Phe Thr Val Lys Val Gly Asp
        35                  40                  45

Lys Glu Leu Phe Thr Asn Arg Trp Asn Leu Gln Ser Leu Leu Leu Ser
    50                  55                  60

Ala Gln Ile Thr Gly Met Thr Val Thr Ile Lys Thr Asn Ala Cys His
65                  70                  75                  80

Asn Gly Gly Gly Phe Ser Glu Val Ile Phe Arg Cys Asn His
                85                  90

<210>  5
<211>  20
<212>  DNA
<213>  artificial

<220>
<223>  CpG oligodeoxynucleotide

<400>  5
tccatgacgt tcctgacgtt                                                   20

<210>  6
<211>  48
<212>  DNA
<213>  artificial

<220>
<223>  PCR primer

<400>  6
ctagctctga aaaggatgaa ctttgagaat tctgactcag aatagctc                    48

<210>  7
<211>  56
<212>  DNA
<213>  artificial

<220>
<223>  PCR primer

<400>  7
cttttcagag ctagtagaat taggatgata gcggccgcta cgaaaaataa cttcgc           56

<210>  8
<211>  17
<212>  DNA
<213>  artificial

<220>
<223>  sShigaATpE primer

<400>  8
cactactacg ttttaac                                                    17


<210>  9
<211>  15
<212>  DNA
<213>  artificial

<220>
<223>  Shiga-fd primer

<400>  9
cggcgcaact atcgg                                                      15


<210>  10
<211>  24
<212>  DNA
<213>  artificial

<220>
<223>  oligonucleotide sulfate 1

<400>  10
ggccgccatc ctaattctac ttct                                            24


<210>  11
<211>  23
<212>  DNA
<213>  artificial

<220>
<223>  oligonucleotide sulfate 2

<400>  11
ctcagaagta gaattaggat ggc                                             23


<210>  12
<211>  28
<212>  DNA
<213>  artificial

<220>
<223>  oligonucleotide sulfate 3

<400>  12
gagtctgaaa aagatgaact ttgatgag                                        28


<210>  13
<211>  37
<212>  DNA

<213>  artificial

<220>
<223>  primer A

<400>  13
agcgaagtta tttttcgttg ttgactcaga atagctc                                37


<210>  14
<211>  33
<212>  DNA
<213>  artificial

<220>
<223>  primer B

<400>  14
gagctattct gagtcaacac gaaaaataac ttc                                    33